# FASCICULE DE BREVET EUROPEEN

(11) **EP 2 083 762 B1**
(45) Date de publication et mention de la délivrance du brevet: **22.06.2016**
(21) Numéro de dépôt: 07858722.7
(22) Date de dépôt: 21.11.2007
(51) Int. Cl.: A61F 2/46

(54) **PORTE PROTHESES ET LEURS APPLICATIONS**
PROTHESENHALTER UND ANWENDUNG DAVON
PROSTHESIS HOLDER AND APPLICATION THEREOF

(30) Priorité: 28.11.2006 FR 0610416
(43) Date de publication de la demande: 05.08.2009
(73) Titulaire: Spineart SA, 1217 Meyrin (CH)
(72) Inventeur: FUHRER, Christophe, 74150 Valliere (FR); LEVIEUX, Jérôme, 1290 Versoix (CH)
(74) Mandataire: Cabinet Plasseraud
(86) Numéro de dépôt international: PCT/FR2007/052374
(87) Numéro de publication internationale: WO 2008/065293

(56) Documents cités:
- WO-A-2006/016384
- WO-A1-2006/065774
- DE-A1- 10 149 385
- US-A- 5 683 464
- US-A1- 2005 119 665
- US-A1- 2005 197 706
- US-A1- 2005 273 171
- US-B1- 6 267 763

## Description

La présente invention concerne des porte prothèses de disques et leurs applications.

Les pathologies du rachis sont de plus en plus traitées par les techniques dites de « non-fusion » et en particulier par l'implantation de prothèses de disques.

Ces prothèses semblent donner de bons résultats.

Cependant, leur implantation est un geste très important et parfois délicat. En fait, la facilité et la précision de l'implantation jouent un rôle prépondérant dans le résultat final, presque autant que les qualités des prothèses de disques elles mêmes.

En effet, la position de la prothèse dans l'espace discal doit être parfaite pour garantir un bon fonctionnement. Le chirurgien s'assure de la position de la prothèse lors de l'intervention chirurgicale à l'aide de rayons X.

Une prothèse de disque est un objet de petite taille, difficile à manipuler (de l'ordre du cm pour sa plus grande dimension et de quelques mm pour son épaisseur). En conséquence son installation met en oeuvre un instrument séparé appelé porte prothèses ou outil d'insertion. Cet instrument doté d'un manche ou poignée est fermement fixé à la prothèse pour permettre au chirurgien de manipuler en force cette dernière. Après installation de la prothèse, il doit pouvoir être retiré et la prothèse laissée en place.

Actuellement, ces instruments sont souvent compliqués. Notamment, le montage de la prothèse sur le porte prothèse peut s'avérer délicat. De plus, ces porte prothèses sont réalisés en matériaux radio opaques, ce qui ne facilite pas la visualisation aux rayons X de la position de la prothèse.

Certains porte prothèses comme celui de la prothèse Cervidisc de la société Scient'X ou de la prothèse Prestige de la société Medtronics, forment une pince venant serrer la prothèse. Lorsque la prothèse est installée dans la bonne position, on la libère en desserrant la pince ou en tirant sur le manche.

Ces instruments fonctionnent bien, cependant, le montage de la prothèse sur son porte prothèse est délicat et doit être réalisé lors de l'intervention par le chirurgien ou le personnel infirmier compétent. Ceci représente une perte de temps et des risques de faute. De plus ces instruments sont radio opaques, généralement métalliques. Or, lorsque la prothèse est en place, le chirurgien contrôle (souvent de profil) sa position pour voir si elle lui convient ou s'il doit encore la déplacer (par exemple l'impacter plus). Ceci est fait par rayons X. Il est donc très difficile au chirurgien de faire la différence entre la prothèse et le porte prothèse; notamment, le chirurgien a du mal à visualiser la position de la face antérieure de la prothèse qui est en contact avec le porte prothèse.

Par ailleurs, quels que soient les systèmes existants (prothèses Maverick de la société Medtronics, Mobi-c de la société LDR, Prodisc de la société Synthes), la déconnexion entre le porte prothèse et la prothèse ne garantit pas la stabilité de l'implant dans l'espace discal. En effet, le chirurgien desserre le porte prothèse puis le retire. Donc, lorsqu'il est desserré il peut se libérer aisément même si la prothèse n'est pas très stable dans l'espace.

De plus, les prothèses de disque étant constituées d'au moins deux pièces mobiles l'une part rapport à l'autre, il n'est pas facile d'installer ces prothèses sur leur porte prothèse lors de l'intervention au bloc opératoire (C'est le cas notamment pour les prothèses Prodisc (Synthes), ou Prestige (Medtronics), ou Cervidisc (Scient'X).

L'objet de l'invention a pour but de remédier aux inconvénients des porte prothèses ci-dessus en proposant un porte prothèses en matériau radio transparent destiné à être fixé d'un côté à la prothèse par un système de pince et possédant un organe d'accouplement réversible à un manche comme un trou fileté ou autre de l'autre côté.

US 5 683 464 A décrit un implant de disque spinal destiné à la fusion de vertèbres adjacentes, comprenant un corps solide ayant quatre faces disposées pour définir un corps solide rectangulaire et deux faces qui définissent les extrémités du corps solide. Un kit qui peut être utilisé par un chirurgien comprend un implant et un outil d'installation d'implant dimensionné de façon à maintenir de manière amovible l'implant. Une paire de bras flexibles opposés s'étendant à partir d'une base, séparés de telle sorte que l'implant peut s'ajuster entre les bras. Le porte-implant peut comprendre une bande de libération cassable s'étendant autour de l'implant pour le maintenir en place jusqu'à ce que le point faible de la bande de libération soit intentionnellement brisé lors de l'implantation.

US 6 267 763 B1 décrit un dispositif destiné à la fusion de vertèbres adjacentes et un outil d'installation et désinstallation comportant deux bras parallèles.

US 2005/197706 A1 décrit une prothèse de disques intervertébraux qui comprend au moins trois pièces comprenant une plaque supérieure, une plaque inférieure et un noyau mobile. Le document décrit également un dispositif d'insertion pour cette prothèse, comprenant plusieurs pièces, à savoir un clip et un chargeur équipé avec une tête dont les dimensions sont adaptées pour recevoir en même temps la prothèse et le clip, le chargeur étant lui-même constitué d'un guide, d'un corps muni d'une tête, et d'un arrêt ajustable.

US 2005/273171 A1 décrit une unité fonctionnelle vertébrale artificielle comprenant un implant intervertébral expansible qui peut être inséré par voie chirurgicale postérieure et est utilisé avec un ou plusieurs dispositifs de remplacement de facette. Des instruments pour l'insertion sont aussi décrits.

Après de longues recherches la demanderesse a mis au point un porte prothèses qui notamment n'interfère pas avec le repérage de la prothèse, est facile à retirer, et peut aussi permettre de s'assurer de la stabilité de la prothèse. De plus, l'accouplement sur la prothèse réalisé au bloc opératoire est très facile (simple vissage par exemple).

C'est pourquoi la présente demande a pour objet un porte prothèses pour prothèse discale caractérisé en ce qu'il est réalisé en matériau radio transparent, en ce qu'il comprend, d'un côté un organe d'accouplement (9) à un manche et du côté opposé, un organe d'accouplement réversible à une prothèse discale comprenant des bras (3,4) en forme de U, en ce que l'organe d'accouplement réversible est agencé pour permettre le désaccouplement par simple traction opérée perpendiculairement à une colonne vertébrale lorsque la prothèse discale est stable entre deux vertèbres, en ce que la force de retenue de l'organe d'accouplement réversible sur la prothèse discale est réglée pour ne pas permettre le désaccouplement par simple traction lorsque la prothèse discale n'est pas stable dans l'espace inter vertébral et en ce qu'il comprend un agencement pour accoupler fermement les pièces mobiles de la prothèse discale, l'agencement pour accoupler fermement les pièces mobiles de la prothèse discale comprenant des rainures ou nervures installées latéralement sur les bras et disposées vers l'intérieur du U.

Ainsi, lorsque la prothèse discale a été correctement impactée entre deux vertèbres et que stabilité est bonne, le désaccouplement intervient par simple traction. Si au contraire la prothèse discale n'a pas été correctement impactée ou si la taille de la prothèse est mal choisie (trop petite) la prothèse n'est pas stable, dans ce cas, lors de la traction opérée par le chirurgien, le retrait de la prothèse discale intervient et le chirurgien comprend que la prothèse discale n'a pas été correctement impactée ou que sa taille n'est pas adaptée à l'espace discal opéré.

Dans la présente demande et dans ce qui suit, le terme « accouplement réversible» désigne un accouplement pouvant être suivi d'un désaccouplement, notamment sans usage d'outil.

L'organe d'accouplement réversible du porte prothèse à une prothèse discale peut prendre de nombreuses formes. De préférence il comprend un dispositif suiveur d'une came prévue sur la prothèse, ledit dispositif suiveur étant monté élastiquement sur le porte prothèse.

Par exemple, le dispositif suiveur est un renflement prévu sur des bras en forme de U du porte prothèses (formant une fourche), lesdits bras étant agencés pour enserrer une prothèse et ledit renflement étant dirigé vers l'intérieur du U. De préférence le renflement est prévu vers l'extrémité des bras en forme de U.

Lors de l'accouplement, le dispositif suiveur est guidé par exemple par une rainure ou nervure constituant le début de la came puis cette rainure ou nervure se creuse et le dispositif suiveur (le renflement) vient se loger dans l'évidement formé pour fermement tenir la prothèse.

De préférence un dispositif suiveur est prévu sur chaque bras, notamment en position symétrique par rapport à la direction de retrait. Avantageusement, quatre dispositifs suiveurs sont prévus.

Dans l'exemple ci-dessus, la profondeur du creux de la came, la longueur, l'épaisseur des bras portant le dispositif suiveur ainsi que leur géométrie (profil en coupe notamment), et le matériau constitutif du porte prothèse conditionnent la force de retenue de l'organe d'accouplement réversible sur la prothèse discale. L'homme de l'art peut avec quelques expériences simples déterminer les paramètres nécessaires pour obtenir la force de retenue souhaitée.

Pour une prothèse à destination cervicale, la force de retenue, nécessaire au déclenchement, peut être de 6 à 30 Newtons, de préférence de 8 à 25 N, notamment de 9 à 20 N, tout particulièrement de 10 à 15 N.

Pour une prothèse à destination lombaire, la force de retenue, nécessaire au déclenchement, peut être de 8 à 50 Newtons, de préférence de 10 à 40 N, notamment de 12 à 30 N, tout particulièrement de 15 à 25 N.

L'homme de l'art comprend que les renflements peuvent être placés en des emplacements variés du porte prothèse aussi longtemps qu'ils sont montés élastiquement et que des évidements correspondants sont prévus sur la prothèse. De même, les renflements peuvent être placés sur la prothèse et les évidements sur le porte prothèse.

L'organe d'accouplement réversible du porte-prothèse à une prothèse discale peut aussi comprendre des tétons, de préférence en forme de cylindres, destinés à être installés dans des trous correspondants prévus à cet effet dans la prothèse. Les tétons peuvent être entaillés et légèrement ouverts afin d'être installés en force et de maintenir la prothèse par adhérence, un effort minimum étant aussi nécessaire pour obtenir le désaccouplement.

Des surfaces de frottement latérales d'un porte prothèse en forme de fourche et de la prothèse peuvent aussi être pratiquement parallèles mais légèrement trapézoïdales (par exemple avec une inclinaison relative de 0,5 à 1° d'angle, la plus courte base étant vers l'ouverture de la fourche. Une palme peut joindre les branches d'un porte prothèse en forme de fourche et un renflement ou un évidement peut être prévu sur la partie palmée.

L'homme de l'art peut comprendre sans effort quand le terme "un" ou "une" signifie "au moins un" ou "au moins une". Par exemple lorsque l'on dit que l'organe d'accouplement réversible du porte prothèse à une prothèse discale peut comprendre "un dispositif suiveur" d'une came prévue sur la prothèse, il faut entendre "au moins un dispositif suiveur".

Dans des conditions préférentielles de mise en oeuvre de l'invention, le porte prothèses ci-dessus comprend un manche et une pièce de retenue séparés ainsi qu'un système d'accouplement réversible du manche à la pièce de retenue.

Le manche permet au chirurgien de saisir le porte prothèse, aussi bien pour la mise en place et l'impaction de la prothèse que pour le désaccouplement et la vérification de la bonne installation de celle-ci.

Le manche peut être accouplé à la pièce de retenue par tout moyen bien connu de l'homme de l'art. On peut citer par exemple les dispositifs par vis et filetage, les dispositifs à baïonnette, à clip etc...

Dans des conditions préférentielles de mise en oeuvre de l'invention, le porte prothèses ci-dessus comprend deux bras formant une pince en forme de U.

Chacun des deux bras comprend de préférence un renflement, ledit renflement étant dirigé vers l'intérieur du U.

Dans d'autres conditions préférentielles de mise en oeuvre de l'invention, le porte prothèses ci-dessus a au maximum la même largeur que la prothèse discale à laquelle il est destiné.

Dans encore d'autres conditions préférentielles de mise en oeuvre de l'invention, le porte prothèses ci-dessus a une longueur, dans la direction du manche, de préférence inférieure à 10 cm, notamment inférieure à 5 cm, particulièrement inférieure à 3 cm, tout particulièrement inférieure à 2 cm. Pour une prothèse à destination cervicale il a une largeur de préférence de 1 à 3 cm, notamment de 1,2 à 2 cm, particulièrement de 1,3 à 1,9 cm, tout particulièrement d'environ 1,5 cm. Pour une prothèse à destination lombaire il a une largeur de préférence de 2 à 6 cm, notamment de 3 à 5 cm, particulièrement de 3 à 4 cm, tout particulièrement d'environ 3,5 cm.

Dans toujours d'autres conditions préférentielles de mise en oeuvre, le porte prothèses de l'invention a au maximum la même hauteur que la prothèse discale à laquelle il est destiné. Notamment il a au maximum la même hauteur et au maximum la même largeur que la prothèse discale à laquelle il est destiné.

Le porte prothèses de l'invention est radio transparent. Il est par exemple réalisé en résine thermoplastique. La résine thermoplastique est par exemple du polyéthylène à haut poids moléculaire, du PEEK chargé en fibres de verre ou carbone ou vierge, des polyphénylsulfones commercialisées sous le nom de Radel® et de préférence de l'acetal (résine acétalique homopolymère thermoplastique) renforcé ou non). Il peut être aussi réalisé en deux ou plus de deux matériaux différents. Dans ce cas, au moins la majeure partie servant à la préhension de la prothèse et en contact avec ladite prothèse est avantageusement radio transparente pour permettre de visualiser la prothèse dans l'espace discal, tandis que le reste du porte prothèses est ou n'est pas réalisé en matériau radio transparent.

Le manche peut être réalisé dans les mêmes matériaux que le porte prothèses, ou en un matériau différent, par exemple un métal comme l'acier inoxydable qui convient parfaitement.

Les porte prothèses objet de la présente invention possèdent de très intéressantes qualités.

La fourchette étant radio transparente, il est aisé de visualiser la prothèse dans l'espace discal. De plus, compte tenu de sa conception, une simple traction suffit à la désolidariser de la prothèse. Ceci a aussi l'avantage de s'assurer de la stabilité de la prothèse, en effet lors de la traction, si la prothèse reste accrochée au porte prothèses, on peut considérer que sa stabilité primaire dans l'espace discal n'était pas suffisante et il faut en général choisir une autre taille, plus grande.

Ils permettent de maintenir solidaires les différentes pièces d'une prothèse discale pendant l'intervention. Ils ont par ailleurs un encombrement réduit puisque leur hauteur peut être inférieure à celle de la prothèse discale et de même, leur largeur peut être inférieure à celle de ladite prothèse discale.

On peut aussi disposer d'une prothèse déjà installée en usine sur le porte prothèse. Le montage du porte-prothèse se résume ainsi au vissage d'une tige dans le trou fileté prévu à cet effet. Ce geste est à l'évidence facile.

C'est pourquoi la présente demande a aussi pour objet un ensemble (ou kit) comprenant un porte-prothèse et une prothèse, de préférence pré montés ainsi que, notamment, un manche. Préférentiellement, les divers éléments de l'ensemble sont stériles et particulièrement stériles sous emballage. Le manche peut ne pas être stérile.

L'agencement en deux parties du porte-prothèse permet de plus d'éviter les manipulations de l'implant qui sont autant de risques de contamination. La prothèse étant montée en usine sur son porte prothèse on peut même monter le manche sur le porte prothèse sans jamais toucher à la prothèse, c'est la technique dite du « no touch » c'est-à-dire « on ne touche rien, qui est la plus sûre sur le plan de l'asepsie.

Ces propriétés et qualités sont illustrées ci-après dans les figures. Elles justifient l'utilisation des porte-prothèses ci-dessus décrits, dans l'installation de prothèses discales entre deux vertèbres.

C'est pourquoi la présente demande a aussi pour objet une méthode d'installation d'une prothèse discale entre deux vertèbres dans laquelle on place entre deux vertèbres une prothèse discale à l'aide d'un porte-prothèses ci-dessus décrit.

La présente demande a encore pour objet une méthode de choix d'une prothèse discale à installer entre deux vertèbres, dans laquelle on place entre deux vertèbres une prothèse discale à l'aide d'un porte-prothèses ci-dessus décrit, on opère une traction sur le porte-prothèses et on observe si la prothèse discale reste en place ou part avec le porte-prothèses.

Les conditions préférentielles de mise en oeuvre des porte-prothèses ci-dessus décrites s'appliquent également aux autres objets de l'invention visés ci-dessus, notamment aux ensembles comprenant un porte-prothèse ci-dessus et une prothèse discale.

L'invention sera mieux comprise si l'on se réfère aux dessins annexés sur lesquels
- la figure 1 représente une vue en perspective d'une prothèse discale constituée d'au moins deux pièces mobiles l'une part rapport à l'autre, avec ses pièces écartées l'une de l'autre.
- la figure 2 représente une vue en perspective d'un porte prothèse discale ainsi que la prothèse correspondante, séparés,
- la figure 3 représente une vue en perspective d'un porte prothèse discale ainsi que la prothèse correspondante, pré montés.

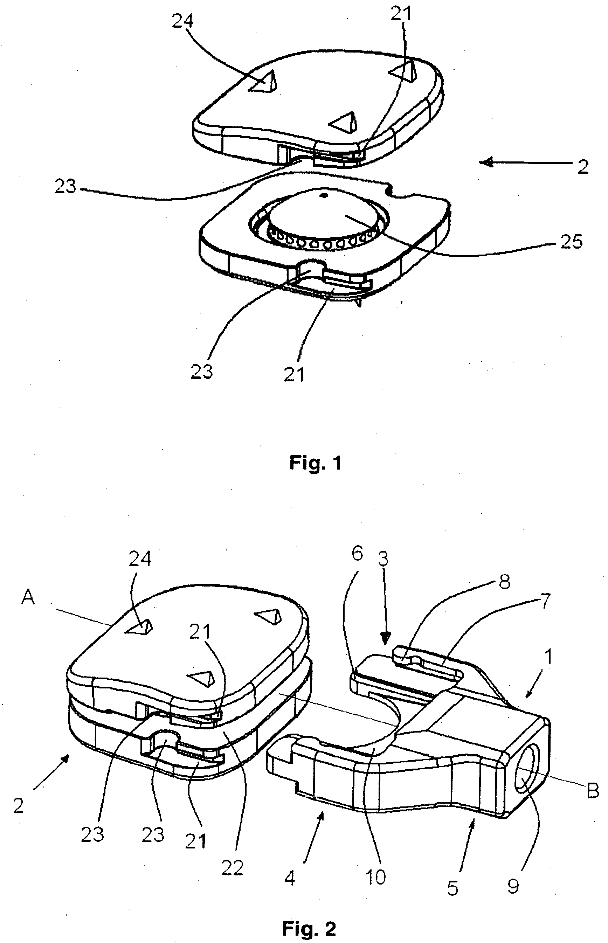

Sur la figure 1 on observe une prothèse discale constituée d'au moins deux pièces mobiles l'une part rapport à l'autre, avec ses pièces écartées l'une de l'autre.

La prothèse 2 comprend un plateau supérieur et un plateau inférieur de forme, vus de dessus, environ carrée à coins arrondis. Chaque plateau de la prothèse 1 a une taille d'environ 1,5 cm sur 1,3 cm sur cette représentation. Le plateau inférieur comprend un évidement dans lequel est logé un dôme 25. Le plateau supérieur comprend un évidement concave dont la forme correspond à celle du dôme.

La prothèse 2 comprend, en creux, les éléments complémentaires à ceux, en relief, du porte prothèse correspondant de la figure 2. Chaque plateau comprend notamment le long de deux côtés opposés, des rainures 21 formant une came. Des évidements 23 au nombre de quatre, deux à gauche et deux à droite de la prothèse c'est-à-dire deux par plateau, sont destinés à recevoir les renflements 8 du porte prothèse. Les dimensions de ces éléments sont adaptées à celles des éléments correspondants du porte prothèse.

La prothèse 2 comprend dessus et dessous des pointes 24 qui permettent l'ancrage de la prothèse après impaction.

Sur la figure 2 on observe un porte prothèse discale 1 ainsi que la prothèse 2 correspondante, séparés. Le porte prothèse 1 est ici représenté sans son manche. Il a une forme générale de fourche en U comprenant deux branches 3, 4 et une partie centrale 5. Chacune des branches comprend trois nervures, une nervure centrale 6, deux nervures secondaires 7 (une seule par branche est visible sur la figure). La nervure centrale 6 d'une forte épaisseur (de 1 à 5 mm selon la taille de la prothèse et sa destination cervicale ou lombaire) sert de guide principal et donne la solidité à l'ensemble, mais ne permet pas l'accouplement réversible selon l'invention. Celui-ci est conféré par les nervures secondaires 7 (d'épaisseur environ 0,4 à 3 mm) qui comprennent à leur extrémité un renflement 8 faisant saillie vers l'intérieur de la fourche. Ce renflement 8 sert de dispositif suiveur. Les renflements 8 sont montés élastiquement grâce à la géométrie (profil en coupe notamment), et au matériau constitutif du porte prothèse 1 ici réalisé en acétal, qui permettent aux deux bras d'être écartés l'un de l'autre.

La coopération des nervures du porte prothèse 1 et de la prothèse 2 empêche l'écartement des pièces de la prothèse 2 et la coopération des renflements 8 et des évidements 23, combinée à la coopération du dôme 25 avec la concavité de la pièce supérieure de la prothèse 2, évite le glissement des pièces de la prothèse 2 l'une sur l'autre, pour accoupler fermement les deux pièces de la prothèse 2.

Dans sa partie centrale 5, le porte prothèses 2 comprend un trou fileté 9 dans lequel on peut visser un manche non représenté.

Une palme 10 relie les deux bras 3 et 4 pour une bonne rigidité.

Le porte prothèse 1 a une hauteur légèrement inférieure à celle de la prothèse discale 2. Il a une longueur, dans la direction du manche, d'environ 1,5 cm, une largeur d'environ 1,5 cm et une hauteur d'environ 6 mm sur cette représentation.

La prothèse 2 est ici représentée avec les deux plateaux rapprochés, le dôme 25 en contact avec l'évidement concave du plateau supérieur.

La prothèse 2 comprend, en creux, les éléments complémentaires à ceux, en relief, du porte prothèses et comprend notamment des rainures 21 formant une came que suit le renflement 8 lors de l'accouplement. La palme 10 vient se loger dans l'espace 22 entre le plateau supérieur de la prothèse et le plateau inférieur. Des évidements 23 au nombre de quatre, deux à gauche et deux à droite de la prothèse sont destinés à recevoir les renflements 8 du porte prothèse. Les dimensions de ces éléments sont adaptées à celles des éléments correspondants du porte prothèse.

La prothèse 2 comprend dessus et dessous des pointes 24 qui permettent l'ancrage de la prothèse après impaction.

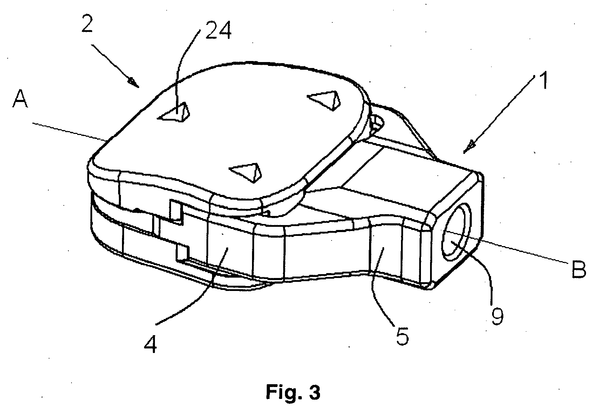

Lors de l'accouplement, représenté à la figure 3, les nervures secondaires 7 viennent s'insérer dans les rainures 21 servant de came correspondantes. En butée, les renflements 8 sont enclenchés dans les évidements 23 pour accoupler fermement les deux pièces de la prothèse 2. L'homme de l'art comprend de l'examen des figures que les renflements 8 peuvent être placés en des emplacements variés du porte prothèse aussi longtemps qu'ils sont montés élastiquement et que des évidements 23 correspondants sont prévus sur la prothèse. De même, les renflements 8 peuvent être placés sur la prothèse et les évidements 23 sur le porte prothèse.

On peut livrer la prothèse 1 avec son porte prothèse 2 déjà installé en usine comme représenté à la figure 3. Le montage final du porte-prothèse se limite ainsi au vissage d'un manche dans le trou fileté 9 prévu dans sa partie centrale 5.

La profondeur du creux 23 des rainures 21, la longueur, l'épaisseur des bras 3,4 portant le dispositif suiveur ainsi que leur géométrie (profil en coupe notamment), et le matériau constitutif du porte prothèses 1 conditionnent la force de retenue du porte-prothèse sur la prothèse.

Pour retirer le porte prothèses 1 en vue de laisser en place la prothèse 2, et vérifier la bonne stabilité de cette dernière, il suffit de tirer sur le manche selon la direction AB. Si les renflements 8 se désenclenchent des évidements 23 en laissant en place la prothèse, cela signifie que cette dernière est bien stable. Les pièces de la prothèse sont libérées et les plateaux peuvent se déplacer l'un par rapport à l'autre, selon la liaison rotule procurée par le dôme.

Sur la figure 3, on observe aussi que l'utilisation de nervures (ou fentes allongées pour la partie femelle) pour accoupler les deux pièces de la prothèse 2 grâce au porte prothèses 1 permet de conférer à l'ensemble un encombrement remarquablement réduit. Le porte prothèses 1 a en effet la même largeur que la prothèse discale 2 comme on peut parfaitement l'observer sur la figure. Il a aussi la même hauteur que la prothèse discale 2. Un encombrement réduit est particulièrement intéressant sur le plan chirurgical.

## Revendications

1. Un ensemble comprenant un porte-prothèse (1) et une prothèse discale (2) constituée d'au moins deux pièces mobiles l'une par rapport à l'autre **caractérisé en ce que** le porte-prothèse est réalisé en matériau radio transparent, **en ce qu'**il comprend, d'un côté un organe d'accouplement (9) à un manche et du côté opposé, un organe d'accouplement réversible à ladite prothèse discale, **en ce que** l'organe d'accouplement réversible est agencé pour permettre le désaccouplement entre le porte-prothèse (1) et la prothèse discale (2) par simple traction opérée perpendiculairement à une colonne vertébrale lorsque la prothèse discale est stable entre deux vertèbres, **en ce que** la force de retenue de l'organe d'accouplement réversible sur la prothèse discale est réglée pour ne pas permettre le désaccouplement par simple traction lorsque la prothèse discale n'est pas stable dans l'espace inter vertébral et **en ce qu'**il est agencé pour accoupler fermement les pièces mobiles de la prothèse discale, et où la prothèse discale est choisie parmi une prothèse à destination cervicale et une prothèse à destination lombaire avec une force de retenue nécessaire au déclenchement comprise entre 6 et 30 Newtons dans le cas d'une prothèse à destination cervicale et avec une force de retenue nécessaire au déclenchement comprise entre 8 et 50 Newtons dans le cas d'une prothèse à destination lombaire.

2. Un ensemble selon la revendication 1, **caractérisé en ce que** l'agencement pour accoupler fermement les pièces mobiles de la prothèse discale comprend des rainures ou nervures (7,21).

3. Un ensemble selon la revendication 1ou 2, **caractérisé en ce que** le porte-prothèse comprend un dispositif suiveur (8) d'une came (21) prévue sur la prothèse, ledit dispositif suiveur (8) étant monté élastiquement sur le porte-prothèse.

4. Un ensemble selon la revendication 3, **caractérisé en ce que** le dispositif suiveur (8) est un renflement prévu sur des bras (3,4) en forme de U du porte-prothèses, lesdits bras (3,4) étant agencés pour enserrer une prothèse discale (2) et ledit renflement (8) étant dirigé vers l'intérieur du U.

5. Un ensemble selon l'une des revendications 1 à 4, **caractérisé en ce que** le porte-prothèse comprend un manche et une pièce de retenue (1) séparés ainsi qu'un système d'accouplement réversible du manche à la pièce de retenue (1).

6. Un ensemble selon l'une des revendications 1 à 5, **caractérisé en ce que** le porte-prothèse est réalisé en résine thermoplastique.

7. Un ensemble selon l'une quelconque des revendications précédentes, **caractérisé en ce qu'**il est pré monté.

## Patentansprüche

1. System, umfassend einen Prothesenträger (1) und eine Bandscheibenprothese (2), welche aus wenigstens zwei zueinander beweglichen Stücken gebildet ist, **dadurch gekennzeichnet, dass** der Prothesenträger aus einem strahlungsdurchlässigen Material gefertigt ist, und dass er an einer Seite ein Kopplungselement (9) an einen Schaft und an der gegenüberliegenden Seite ein lösbares Kopplungselement an die Bandscheibenprothese umfasst, dass das lösbare Kopplungselement dazu eingerichtet ist, das Entkoppeln zwischen dem Prothesenträger (1) und der Bandscheibenprothese (2) durch einfaches Ziehen zu erlauben, welches senkrecht zu einer Wirbelsäule durchgeführt wird, wenn die Bandscheibenprothese sich stabil zwischen zwei Wirbeln befindet, und dass die Haltekraft des lösbaren Kopplungselements an der Bandscheibenprothese eingestellt ist, um das Entkoppeln durch einfaches Ziehen nicht zu erlauben, wenn die Bandscheibenprothese sich nicht stabil in dem Raum zwischen den Wirbeln befindet, und dass es dazu eingerichtet ist, die beweglichen Teile der Bandscheibenprothese fest zu verbinden, und wobei die Bandscheibenprothese aus einer Prothese mit einem zervikalen Ziel und einer Prothese mit einem lumbaren Ziel ausgewählt ist, wobei eine zum Auslösen notwendige Haltekraft zwischen 6 und 30 Newton im Fall einer Prothese mit zervikalem Ziel beträgt, und eine zum Auslösen notwendige Haltekraft zwischen 8 und 50 Newton im Fall einer Prothese mit lumbarem Ziel beträgt.

2. System nach Anspruch 1, **dadurch gekennzeichnet, dass** die Einrichtung zum festen Verbinden der beweglichen Teile der Bandscheibenprothese Nuten oder Rippen (7, 21) umfasst.

3. System nach Anspruch 1 oder 2, **dadurch gekennzeichnet, dass** der Prothesenträger eine Folgevorrichtung (8) eines Nockens (21) umfasst, welcher an der Prothese vorgesehen ist, wobei die Folgevorrichtung (8) elastisch an dem Prothesenträger montiert ist.

4. System nach Anspruch 3, **dadurch gekennzeichnet, dass** die Folgevorrichtung (8) eine Verdickung ist, welche an in U-Form gebildeten Armen (3, 4) des Prothesenträgers vorgesehen ist, welche Arme (3, 4) dazu eingerichtet sind, eine Bandscheibenprothese (2) zu Umspannen, und wobei die Verdickung (8) in Richtung des Inneren des U gerichtet ist.

5. System nach einem der Ansprüche 1 bis 4, **dadurch gekennzeichnet, dass** der Prothesenträger einen Schaft und ein davon getrenntes Rückhalteteil (1) umfasst, sowie ein System zum lösbaren Koppeln des Schafts mit dem Rückhalteteil (1).

6. System nach einem der Ansprüche 1 bis 5, **dadurch gekennzeichnet, dass** der Prothesenträger aus thermoplastischem Harz gefertigt ist.

7. System nach einem der vorhergehenden Ansprüche **dadurch gekennzeichnet, dass** es vormontiert ist.

## Claims

1. An assembly comprising a prosthesis holder (1) and a disc prosthesis (2) constituted by at least two parts which are mobile in relation to each other **characterized in that** the prosthesis holder is made of radiolucent material, **in that** it comprises, on one side, an element (9) for coupling to a sleeve and, on the other side, an element for reversible coupling to the said disc prosthesis, **in that** the reversible coupling element is arranged in order to allow decoupling between the prosthesis holder (1) and the disc prosthesis (2) by simple traction exerted perpendicularly to a spinal column when the disc prosthesis is stable between two vertebrae, **in that** the retention force of the reversible coupling element on the disc prosthesis is adjusted so as not to allow decoupling by simple traction when the disc prosthesis is not stable in the intervertebral space and **in that** it is arranged so as to firmly couple the mobile parts of the disc prosthesis, and wherein disc prosthesis is chosen within a prosthesis intended for the cervical region and a prosthesis intended for the lumbar region, with a retention force, necessary for disengagement, comprised between 6 to 30 Newton for a prosthesis intended for the cervical region and a retention force, necessary for disengagement, comprised between 8 to 50 Newton for a prosthesis intended for the lumbar region.

2. An assembly according to claim 1, **characterized in that** the arrangement for firmly coupling the mobile parts of the disc prosthesis comprise grooves or ribs (7, 21).

3. An assembly according to claim 1 or 2, **characterized in that** the prosthesis holder comprises a follower device (8) of a cam (21) provided on the prosthesis, said follower device (8) being elastically mounted on the prosthesis holder.

4. An assembly according to claim 3, **characterized in that** the follower device (8) is a bulge provided on U-shaped arms (3, 4) of the prosthesis holder, said arms (3, 4) being arranged in order to grip a disc prosthesis (2) and said bulge (8) being directed towards the inside of the U.

5. An assembly according to one of claims 1 to 4, **characterized in that** the prosthesis holder comprises a separate sleeve and retaining part (1) as well as a system for the reversible coupling of the sleeve to the retaining part (1).

6. An assembly according to one of claims 1 to 5, **characterized in that** the prosthesis holder it is made of thermoplastic resin.

7. An assembly according to any of preceding claims, **characterized in that** it is pre-mounted.
